Europäisches Patentamt

European Patent Office

Office européen des brevets

(19)

(11) Publication number : **0 108 301 B2**

## NEW EUROPEAN PATENT SPECIFICATION

(12)

(45) Date of publication of the new patent specification : **01.09.93 Bulletin 93/35**

(21) Application number : **83110394.0**

(22) Date of filing : **19.10.83**

(51) Int. Cl.$^5$ : **C12N 15/56, C12N 15/57, C12N 15/75, C12N 9/28, C12N 9/34, C12N 9/54, C12R 1/07**

(54) Method of heterologous cloning of gene in bacillus microorganism.

(30) Priority : **01.11.82 US 438544**

(43) Date of publication of application :
**16.05.84 Bulletin 84/20**

(45) Publication of the grant of the patent :
**23.11.89 Bulletin 89/47**

(45) Mention of the opposition decision :
**01.09.93 Bulletin 93/35**

(84) Designated Contracting States :
**AT BE CH DE FR GB IT LI NL SE**

(56) References cited :
**EP-A- 0 021 468
EP-A- 0 034 470
EP-A-00 579 76
GB-A- 2 091 268
THE EMBO JOURNAL, vol. 1, no. 7, 1982, pages 791-799, IRL Press Ltd., Oxford, GB; A.KLIER et al.: "Cloning and expression of the crystal protein genes from Bacillus thuringiensis strain Berliner 1715"
GENE, vol. 12, 1980, pages 147-154, Elsevier/North-Holland Biomedical Press; B.MICHEL et al.: "DNA cloning in Bacillus subtilis. III. Efficiency of random-segment cloning and insertional inactivation vectors"
CHEMICAL ABSTRACTS, vol. 99, no. 7, August 1983, page 114, no. 48471a, Columbus, Ohio, US; J.BAL et al.: "Construction of plasmid vectors for gene cloning in Escherichia coli and Bacillus subtilis" & ACTA MICROBIOL. POL. 1982, 31(3-4), 217-25**

(56) References cited :
**Kreft et al., Molec.gen. Genet. vol. 162, 1978, pp. 59-67
Ehrlich: Proc. Natl. Acad. Sci. USA, vol. 75, 1978, pp. 1433-1436
Changand Cohen, Molec. Gen. Gent., vol. 168, 1979, pp. 111-115
Maniatis et al.,"Molecular Cloning, A Laboratory Manual". Cold Spring Harbor, N.Y.1982, pages 316-317
Wilhelmand Hollenberg, Nucleid Acids Research, vol. 13, no. 15, 1985, pp. 5717-5722
Gerhartz in "Enzymes in Industry", 1990, VCH Verlags.GmbH, Weinheim, FRG**

(73) Proprietor : **SOLVAY ENZYMES, INC. (a Delaware corporation)
3333, Richmond Avenue
Houston, Texas, 77098 (US)**

(72) Inventor : **Grosch, Josephine
57113 (Rt.7) Decamp-B
Elkhart Indiana 46514 (US)**
Inventor : **Wilson, Gary A.
54401 Susquehanna
Elkhart Indiana 46514 (US)**
Inventor : **Wollweber, Karen
340 Becado Circle, Nr. 28
Mishawaka Indiana 46514 (US)**
Inventor : **Yehle, Clifford O.
23215 Kingsland Court
Elkhart Indiana 46514 (US)**

(74) Representative : **Meyers, Liliane et al
Solvay & Cie S.A. Département de la propriété industrielle 310, rue de Ransbeek
B-1120 Bruxelles (BE)**

EP 0 108 301 B2

# Beschreibung

## Background of the invention

### 1. Field of the invention

Microbial enzymes are becoming increasingly important in such diverse fluids as medicine, agriculture and organic chemicals production. The genus *Bacillus* comprises a group of bacteria that can be easily maintained and cultivated, yet are markedly heterogeneous in character. All of the 48 species of the *Bacillus* genus listed in *Bergey's Manual of Determinative Bacteriology* secrete soluble enzymes.

Classical genetic manipulation techniques, e.g., ultraviolet irradiation and mutation selection, have been used to improve the yield and properties of enzymes such as α-amylase. In the 1960's molecular biologists utilized bacteria, mainly *Escherichi coli* and the viruses that infect them, deciphered the genetic code, established the molecular basis of mutation, discovered methods by which deoxyribonucleic acid (DNA) could be passed from one cell to another, and elucidated in precise detail the manner in which a living microorganism can control the expression or activity of specific genes. This information was utilized in the 1970's to form the basis of the genetic engineering industry, involving recombinant DNA technology.

Recombinant DNA technology involves the transfer of genetic material (genes or DNA fragments) from one microorganism into a second microorganism and subsequent propagation of the combined genetic material.

The specific gene, i.e., DNA sequence, carrying the desired genetic trait, can be either isolated by physico-chemical procedures or chemically synthesized. This DNA sequence is then covalently attached to a small circular piece of DNA called a plasmid. The design of the plasmid molecule is such that it can be opened at a specifc point by cleavage with a class of molecular enzymes, i.e., restriction endonucleases, and the "foreign" piece of DNA inserted to produce a recombinant plasmid. Successful recombinant DNA techniques require that the DNA be inserted at a site on the plasmid that allows genetic activity in a host microorganism.

One area of interest in recombinant DNA techniques involves cloning genes encoding enzymes such as α-amylase, protease, amyloglucosidase and glucose isomerase. The structure of these enzymes and the genetic system of the microorganisms which produce such enzymes have not been extensively characterized.

Because no ability to isolate such genes of interest and no technique to allow direct selection for their presence or the presence of the gene prdoucts, i.e., the enzymes of interest, exists, "shot-gun" cloning, i.e., cloning of the entire genome of the organism of interest, is often necessary.

For various reasons, "shot-gun" cloning dierctly into a *Bacillus* host, such as *B. subtilis* is impractical. Some reasons include: scarcity of desirable *B. subtilis* cloning vectors (plasmids), inefficiency of transformation, and very limited transformation capability of recombinant plasmid molecules from a ligation mixture. Therefore, a cloning strategy must be carefully chosen in order to maximise the probaility of cloning and identifying the desired gene in a minimum number of steps.

## Prior art

It is known that genes encoding or regulating α-amylase in a *Bacillus* strain can be introduced into another *Bacillus* strain if the two strains are sufficeintly closely related, i.e., if there is extensive genetic homology between the two strains. This is referred to as homologous transformation. For example, *J. Bacteriol., 120*: 1114-1150 (1974) describes the introduction of DNA from *B. subtilis* var *amylosacchariticus* having exceptionally high α-amylase activity, into a genetically similar (homogolous) microorganism having relatively low α-amylase activity (*B. subtilis* 168 Marburg). The transformed microorganisms which were produced acquired high α-amylase activity.

However, most *Bacillus* species are not sufficiently related to *B. subtilis*, i.e., are not sufficiently homologous, to permit the DNA obtained from one *Bacillus* strain to be efficiently introduced into a different *Bacillus* strain. *J. Bacteriol., 111*, 705-716 (1972).

*Appl. Environ. Microbiol., 39*: 274-276 (1980) established that the effect of incorporating related genes for *a*-amylase production and protease into a strain produced an increase in α-amylase production of a synergistic nature. The overall approach involved the stepwise introduction of the genes into a recipient *B. subtilis* Marburg 6160 microorganism by a stepwise transformation procedure. The authors indicate that because the transformation procedure requires chromosomal homology, a suggested alternative approach which can utilize chromosomal heterology would involve the development of vectors for cloning the genes and introducing them into a modified recipient, i.e., a "mother cell" in a more purified form.

The literature indicates that it is difficult to use conventional transformation techniques in a heterologous transformation, e.g., to introduce genes from *B. amyloliquefaciens* into *B. subtilis*. As indicated in *J. Bacteriol., 111*: 705-716 (1972), particularly Table 2, transformation of a *B. subtilis* 168 strain (BR151) with DNA from a homologous *B. subtilis* 168 strain produced one thousand fold more transformants for three tested loci than DNA from a heterologous source. *B. amyloliquefaciens H.*

The closest prior art appears to be that described by Rapoport, et. al. in *Mol. Gen. Genet., 176*: 239245 (1979) and Palva, et. al. in *Gene, 15*: 43-51 (1981).

Rapoport et. al. describes the use of a bifunctional plasmid (pHV33) to construct a colony bank of *E. coli*-containing plasmids with DNA fragments corresponding to the entire genome of *B. subtilis*. Rapoport et al. involved a homologous cloning of DNA from *Bacillus subtilis* into a second host *Bacillus subtilis* of the same species. Rapoport et. al. obtained large fragments of DNA obtained by mechanical shearing and indicated that complete digestion with a restriction endonuclease is undesirable because it may cause loss of genetic activity by cleavage of DNA within the desired structural gene.

Rapoport identified the recombinant clones by complementation of auxotrophic mutations of certain *B. subtilis* host strains. This method utilizes direct selection techniques to identify the presence of the desired gene or gene product. By this approach Rapoport was limited to searching only for clone genes which coded for nutritional requirements of *B. subtilis* mutants. Cloned genes coding for the enzymes mentioned above could not be selected and identifed by this approach.

Palva et. al., describes the cloning of the α-amylase gene from *B. amyloliquifaciens* into a plasmid vector puB110. This work involved heterologous cloning into *B. subtilis* as a host microorganism, but the vector used was not bifunctional.

A. Klier et al published an article in THE EMBO JOURNAL, Vol. 1, No. 7, 1982, pages 791-799, entitled "Cloning and expression of the crystal protein genes from *Bacillus thuringiensis* strain Berliner 1715" which discloses a procedure which utilizes insertional inactivation.

An insertional inactivation is also disclosed by B. Michel et al., in GENE, Vol. 12, 1980, pages 147-154, Elsevier/North-Holland Biomedical Press entitled "DNA cloning in *Bacillus subtilis*". III. Efficiency of random-segment cloning and insertional inactivation vectors".

None of the references discussed above describes the method of the present invention to achieve heterologous cloning of a gene encoding α-amylase, protease, amyloglucosidase or glucose isomerase.

## Summary of the invention

The present invention is directed to a method for cloning and isolating a gene encoding an enzyme selected from the group consisting of α-amylase, protease, amyloglucosidase from a *Bacillus* microorganism into a *Bacillus* host microorganism of another species which comprises the steps of:

(a) digesting a bifunctional plasmid having the capability to replicate in both *E. coli* and *B. subtilis*, wherein said plasmid contains at least two selectable markers, with an endonuclease to produce a linear plasmid molecule;

(b) generating DNA fragments containing said gene and incorporating said DNA fragments into said plasmid of (a) such that insertional inactivation of one of said selectable markers to produce a non-functional marker occurs, leaving at least one functional selectable marker, to produce a mixture containing recombinant hybrid plasmids and as a by-product, said bifunctional plasmids of (a);

(c) incorporating said mixture of (b) into *E. coli* microorganisms which do not have said selectable markers of (a) to transform said *E. coli*,

(d) selecting *E. coli* transformants containing said plasmids of (b) by means of said functional selectable marker;

(e) identifying transformants containing said recombinant plasmids by screening said transformants of (d) for the presence of said nonfunctional insertionally-inactivated marker;

(f) extracting plasmid DNA from said *E. coli* transformants of (e);

(g) transforming said *Bacillus* host microorganisms which do not contain said selectable markers of (a) with said plasmid DNA from (f);

(h) selecting said *Bacillus* host transformants containing recombinant plasmids by means of said functional selectable marker;

(i) screening said *Bacillus* host microorganisms selected in step (h) by overlaying a sterile semipermeable membrane on the surface of an agar medium containing a substance which is capable of being reacted upon by said enzyme, growing said transformed microorganisms on said membrane and allowing said enzyme to pass into said agar while retaining said transformed microorganisms on said membrane, removing said membrane from said agar surface, and contacting said agar with a substance which allows detection of whether a reaction between the said enzyme and the said substance has taken place; and

(j) isolating therefrom a *Bacillus* host microorganism capable of producing said enzyme encoded by said gene on said plasmid.

## Detailed description of the invention

The method of the present invention provides a pathway for the heterologous cloning of a gene encoding α-amylase, protease, amyloglucosidase or glucose isomerase enzymes, into a *Bacillus* host microorganism. A single gene or multiple gene copies can be cloned by the present method.

In the following description, various microorganisms are designated as having an ATCC or BGSC number. Each ATCC designated microorganism is available from the American Type Culture Collection, Rockville, Maryland, each BGSC designated microorganism is available from the Bacillus Genetic Stock Center, Ohio State University, Columbus, Ohio.

The present invention can be more easily understood by means of the following illustration of insertion of a foreign DNA fragment into a plasmid DNA molecule.

First, a bifunctional plasmid which replicates in both *E. coli* and *B. subtilis*, having the following characteristics, is chosen. The plasmid encodes the genes for ampicillin resistance ($Ap^R$), tetracycline resistance ($Tc^R$), and chloramphenicol resistance ($Cm^R$), such that the $Ap^R$, $Tc^R$, and $Cm^R$ all function in *E. coli*, and only $Cm^R$ functions in *B. subtilis*. These genes function as selectable markers.

The plasmid molecules are digested with a restriction endonuclease which cuts at a specific site within the $Tc^R$ gene. Such digestion results in linear plasmid molecules. These linear molecules are now mixed with the foreign DNA fragments, which have also been produced by digestion with the same restriction endonuclease. The foreign DNA molecules and the linear plasmid DNA moleucles have the same "sticky ends" and these ends will anneal to form recombinant hybrid molecules. The annealed ends are now "sealed" by the action of DNA lignase enzyme.

The ligation reaction produces a mixture of plasmid DNA molecules because the generation of the recombinant molecules is an inefficient process. The plasmid molecules can be of the following types: (a) the original bifunctional plasmid which has not reacted with any foreign DNA fragments, of the phenotype $Ap^R$, $Tc^R$, $Cm^R$, and (b) recombinant hybrid plasmids containing a foreign DNA fragment in the $Tc^R$ site, of the phenotype $Ap^R$, $Tc^S$ (tetracycline sensitive), $Cm^R$. Thus, the tetracycline gene is rendered inactive by the insertion of the foreign DNA fragment and becomes a nonfunctional selectable marker.

The mixture of plasmid molecules described above is incorporated by transformation into *E. coli* microorganisms which initially do not have these markers, i.e., $Ap^S$ (ampicillin sensitive), $Tc^S$. The *E. coli* cells which now contain a plasmid moelcule of either type (a) or (b) described above, can be selected from the cells which have not been transformed (transformation being an inefficient process), by their $Ap^R$ phenotype. The selection is done by growing all the cells on medium containing ampicillin. Only the $Ap^R$ cells will grow.

The *E. coli* cells which contain recombinant hybrid plasmids [(b) in the above discussion] as contrasted with these which contain the original plasmid [(a) in the above discussion] are obtained by screening the $Ap^R$ transformants for the $Tc^S$ phenotype.

The screening is done by growing all the $Ap^R$ cells on medium containing tetracycline. The $Tc^S$ cells will not grow on this medium, but can be recovered from a replicate set of cells grown on medium containing ampicillin (replica-plating).

The plasmid DNA is extracted from these $Ap^R$, $Tc^S$ *E. coli* cells and incorporated into *B. subtilis* host cells, initially not having the previously described markers, by transformation. The *B. subtilis* cells which contain plasmids, are selected from those which do not contain plasmids, by their $Cm^R$ phenotype.

This selection is done by growing all the *B. subtilis* cells on medium containing chloramphenicol. Only the cells which are $Cm^R$ will grow. All of these $Cm^R$ cells contain recombinant hybrid plasmids.

Suitable pasmids are plasmids which are bifunctional, i.e., are capable of replicating in two bacterial species, such as in *Bacillus subtilis* and *E. coli* microorganisms. Such bifunctional plasmids include pHV33, ATCC 39217 and pHV11, described in *Proc. Natl. Acad. Sci.* USA, 75(3): 1433-1436 (1978).

Such plasmids must contain at least two "selectable" markers. Having a selectable marker enables the use of a primary selection technique, e.g., if a transformed microorganism is resistant to a certain antibiotic, the microorganism can be cultured in the presence of such antibiotic and the microorganisms which survive can be selected as viable transformants. Selectable markers are not limited to antibiotic resistance but can include growth requirements, e.g., a microorganism can require the presence of an amino acid such as threonine, lysine, tyrosine, alanine, leucine, or serine. Purines and pyrimidines, such as adenine, thymine, guanine, cytosine and uracil, can also function as a growth requirement and as a selectable marker.

It is also a requirement that one or more of the selectable markers is capable of being "insertionally-inactivated", i.e., insertion of the DNA fragment into the plasmid will inactivate one of the selectable markers. For exmaple, if one of the markers involves a gene which confers drug resistance, such as chloramphenicol resistance ($Cm^R$), ampicillin ($Ap^R$) and tetracycline resistance ($Tc^R$), one of the markers, i.e., the gene, can be inactivated such that the cell is rendered sensitive to the drug, i.e., $Cm^S$, $Ap^S$, or $Tc^S$ , in order to serve as a marker.

The markers can both be the same type, i.e., two antibiotic markers or two growth requirements, or can be a combination of an antibiotic marker and a growth requirement.

For example, a bifunctional plasmid containing a leucine gene and a gene for antibiotic resistance can be used as follows. Insertion of a DNA fragment into the antibiotic-resistant gene could be used to inactivate the antibiotic-resistant gene to produce a nonfunctional marker, leaving the leucine gene as a functional selectable marker. The recombinant plasmids produces are incorporated into a suitable *E. coli* microorganism (described below) which requires leucine for growth, *E. coli* transformants containing both recombinant plasmids and the original bifunctional plasmid are selected by utilizing the leucine growth requirement as a functional selectable marker. The selection is accomplished by growing the microorganisms in the absence of leucine and selecting the microorganisms which survive. From these microorganisms, the *E. coli* transformants containing only the recombinant plasmids are identified by screening the transformants for those which contain a plasmid with the nonfunctional insertionally-inactivated antibiotic resistance marker. The screening is accomplished by identifying those transformants which are unable to grow in the presence of the antibiotic, by use of replica-plating. The plasmid DNA from these transformants is then extracted and used to transform *Bacillus* host microorganisms which do not contain the selectable marker. The desired *B. subtilis* transformants which contain recombinant plasmids are selected by using the functional selectable marker, i.e., the leucine growth requirement, and the desired enzyme producing microorganism is isolated.

As indicated hereinbefore, suitable *Bacillus* host microorganisms are used. A requirement of these microorganisms is that they must not have the selectable markers of the bifunctional plasmid. Suitable *Bacillus* host microorganisms include *Bacillus subtilis* 168 (ATCC 27689), *B. subtilis* (BGSC 1A289), *B. amyloliquifaciens H* (BGSC 10A2), and *B. licheniformis* ATCC 27811.

Similarly, suitable *E. coli* microorganisms can be selected from those which do not have the selectable markers of the bifunctional plasmid. For example, *E. coli* K12 (ATCC 10798) and *E. coli* C600 (ATCC 33525) can be used.

Chromosomal DNA from a suitable *Bacillus* species, containing a desired gene encoding for the desired enzyme, can be isolated by the conventional technique described hereinafter. Suitable microorganisms from which chromosomal DNA can be obtained include the following: α-amylase, *B. cereus* ATCC 21768, protease, *B. amyloliquefaciens* ATCC 23842 and 23844, *B. alkalophilus* ATCC 21522 and *B. licheniformis* ATCC 21424; amyloglucosidase, *B. subtilis* ATCC 31068, and glucose isomerase, *B. licheniformis* ATCC 31667, (as disclosed in U.S. Patent No. 4,355,103).

The DNA fragments can be generated by conventional techniques involving cleavage with an appropriate restriction enzyme, e.g., *Bam HI* or *Sal I*. The DNA fragments should be of a size to ensure that at least one fragment contains the entire gene to be cloned. For the enzymes referred to hereinbefore, α-amylase, protease, amyloglucosidase and glucose isomerase, the fragments should be about 2 megadaltons (Md) or greater in size.

The DNA is incorporated into the bifunctional plasmid by conventional ligation techniques, involving the use of T4 DNA ligase or *E. coli* DNA ligase.

Example I

A. Isolation and purification of *B. licheniformis* DNA

A suitable *B. licheniformis* microorganism containing a gene encoding α-amylase was grown in one liter of Penassay broth (Antibiotic Medium #3, commerically available from Difco Laboratories, Detroit, Michigan) for 18 hours with aeration. Cells were harvested by centrifugation and resuspended in 50 ml of a buffer consisting of 0.15 M NaCl, 0.1 M ethylenediaminetetraacetic acid (EDTA) at pH 8.0. Lysis of the cells was accomplished by the addition of 50 mg lysozyme (egg white crystalline lysozyme, commercially available from Miles Laboratories, Elkhart, Indiana), followed by incubation for 30 minutes at 37°C. Addition of 150 ml of a buffer consisting of 0.1 M tris(hydroxymethyl)aminomethanehydrochloride (tris), 0.1 M NaCl, 0.5 percent sodium dodecyl sulfate (SDS), at pH 8.0, completed the lysis step. Protein was removed by extracting the lysate with 200 ml phenol (which had been equilibrated with 0.1 M tris, pH 8.0) and 20 ml chloroform-isoamylalcohol in a ratio of 24:1. The upper phase was extracted twice more with chloroform-isoamylalcohol. The upper phase was made 0.1 M in NaCl and the DNA was precipitated with two volumes cold 95 percent ethanol. The DNA was dissolved in 150 ml of 0.1 M standard saline citrate buffer (SSC) which consisted of 0.15 M NaCl and 0.015 M sodium citrate, pH 7.0. This DNA solution was treated with 10 mg of RNase for 2 hours at 37°C and then with 1.5 mg protease (Pronase, commercially available from Calbiochem, LaJolla, California) for 30 minutes at 37°C. The solution was extracted twice with phenol (described above), and dissolved in 0.1X SSC. This DNA solution was dialyzed against 0.1 X SSC at 4°C to remove residual phenol, SDS, and ethanol.

The DNA was cleaved with *Sal I* restriction endonuclease (commercially available from Miles Laboratories, Inc. Elkhart, Indiana) under the conditions recommended by the supplier. To ensure that the α-amylase gene was not cleaved by this enzyme, conditions of enzyme treatment were established such that only partial digestion was achieved. The conditions involved using low concentrations of the enzyme over a limited time period. These conditions can be easily determined by one skilled in the art. The resulting fragments were separated by sucrose density gradient centrifugation, and fractions containing DNA fragments 2 megadaltons or greater were selected for cloning.

## B. Isolation and preparation of pHV33 Plasmid

Plasmid pHV33, is available from ATCC in *E. coli* RR1 (ATCC 39217). The plasmid DNA was isolated according to the following procedure. [See *Practical Methods in Molecular Biology*, Robert F. Schleif, (1981)].

The above *E. coli* containing pHV33 was grown in 600 ml of Penassay broth containing 50 μg/ml ampicillin with aeration to mid-exponental growth phase, approximately 3 hrs. At this time, 150 mg of chloramphenicol was added to the culture to allow amplification of plasmid DNA. Incubation was continued for 23 hours. Cells were harvested by centrifugation and washed once in a buffer composed of 0.01 M tris, 0.001 M ethylenediaminetetracetic acid (EDTA), pH. 8.0 (TE buffer). The washed cells were suspended in 5.0 ml of a buffer containing 25 percent sucrose, 0.05 M Tris-HCl, pH 8.0. Lysis was accomplished by the addition of 5 mg of lysozyme followed by incubation in an ice bath for ten minutes. One ml of 0.25 M EDTA solution, pH 8.0, was added and the incubation continued for 10 more minutes. Lysis was completed by the addition of 3.2 ml of a detergent solution composed of the following: 1.0 percent of polyoxyethylene lauryl ether, available from Fisher Scientific Company, Fairhaven, New Jersey under the trade designation Brij 35; 0.4 percent sodium deoxycholate, 0.06 M EDTA, and 0.05 M .Tris-HCl, pH 8.0. The lysis mixture was gently with a glass stirring rod. The lystate was cleared by centrifugation to remove cellular debris and chromosomal DNA. The cleared lysate was subjected to isopycnic cesium chloride density gradient centrifugation in the presence of ethidium bromide to separate covalently closed circular plasmid DNA from other nucleic acids. Cesium chloride was added to the lysate at ambient temperature to a final refractive index of 1.3995. After centrifugation for 40 hours at 130,000×g, the plasmid DNA band was removed, and extracted 6 times with water-saturated sec-butanol to remove ethidium bromide. The upper phase from the last extraction was made 0.3 M in sodium acetate and precipitated with 2 volumes cold ethanol overnight at -20°C. Plasmid DNA was dissolved in TE buffer and dialyzed extensively against TE buffer at 4°C.

The plasmid DNA was then cleaved by *Sal I* restriction endonuclease (for which it contains a unique site) under appropriate conditions such that the circular molecules were converted to linear forms.

## C. Ligation procedure

The linearized plasmid DNA and the *B.*

*licheniformis* DNA fragments were mixed together in the manner described below to produce a collection of circular DNA molecules, each containing pHV33 DNA and somes fragment of the *B. licheniformis* genome.

A reaction mixture consisting of 10.0 μg *B. licheniformis* DNA fragments (treated with *Sal I*), 2.8 μg of *Sal I*-treated pHV33, 0.002 M adenosine triphosphate (ATP), 0.5 μg of T4 DNA ligase (commercially available from Miles Laboratories, Inc.) in an appropriate buffer was incubated at 14.5°C for 18 hours. The total volume of the reaction mixture was 80 μl.

Such a reaction mixture includes the following: re-circularized pHV33 molecules which have not reacted with *B. licheniformis* DNA fragments. (Ap$^R$, Tc$^R$); and recombinant plasmids containing a *B. licheniformis* DNA fragment inserted within the tetracycline gene (Ap$^R$, Tc$^S$).

## D. Transformation of *E. coli* with hybrid plasmids

Sixty μl of the ligation mixture were mixed with μl of 0.3 M CaCl$_2$ and 30 μl H$_2$O. The *E. coli* RR1 cells were previously rendered competent by treatment with CaCl$_2$ and stored at -70°C. The ligation solution was added to 200 μl of thawed *E. coli* cells, and the resulting mixture was heated at 42°C for one minute to complete the transformation. Details of this procedure for transformation of *E. coli* are given in *J. Mol. Biol.*, 52: 159-163, 1970.

The mixture was then diluted with 2.0 ml Penassay broth. This suspension was incubated for 2 hours at 37°C to allow expression of the plasmid-encoded ampicillin resistance gene. The cells were then plated onto TBAB (Tryptose Blood Agar Base, available from Difco Laboratories. Detroit, Michigan) containing 50 μg/ml ampicillin. Only cells which had been transformed by the plasmid DNA grew in this initial selection.

The resulting transformants were subjected to screening on TBAB plates containing 20 μg/ml tetracycline, by replica-plating. Cells which are sensitive to tetracycline harbor a plasmid which contains *B. licheniformis* DNA inserted in the gene for tetracycline resistance, thereby inactivating this gene. In this manner, only clones which harbored a plasmid having the phenotype Ap$^R$, Tc$^S$ were identified and saved.

The following formula was used to calculate the number of clones needed to ensure a 99 percent probability that the entire *B. licheniformis* genome was represented in the clones obtained [See *J. Mol. Biol.* 53: 159 (1970)].

$$P=1-(1-F)^N$$

$$N=1n(1-P)/1n(1-F)$$

For 99% probability

Given: 3Md fragment size

$3×10^9$ dalton=*B. licheniformis* genome

size,

$$N = 4,600$$

Where

F=the fraction of the total genome represented by each fragment and

P=is the probability that a unique DNA sequence is present in the collection of N transformant colonies.

A total of 5,595 clones were identified as being $Amp^R$, $Tc^S$. The 5,595 clones were grown individually in broth cultures, pooled into sets of 30 and the plasmid DNA isolated from each set. Isolation of plasmid DNA was accomplished by detergent lysis followed by ethanol precipitation [See *Proc. Nat. Acad. Sci. USA.* 62:1159-1166 (1969)].

The plasmid pools were then used as donor DNA to transform competent host *B. subtilis* cells which did not contain the selectable markers, i.e., $Amy^-$, $Cm^S$ cells, to $Cm^R$ and the $Cm^R$ transformants were screened for the ability to produce α-amylase ($Amy^+$) as described in section E hereinafter. Transformation of competent *B. subtilis* cells with plasmid DAN is described in *Moelcular and General Genetics, 167*: 251-258 (1979).

Alternatively, for strains of *Bacillus* which are not naturally competent, a protoplast transformation procedure can be used. The precedure involves treatment of the microorganisms with lysozyme to form protoplasts followed by polyethylene glycol-mediated uptake of DNA. [See *Genetic Exchange, Genetic and Cellular Technology*, Vol I, 97-105 (1982)].

E. Screening procedure for $Cm^R$, $Amy^+$ *B. subtilis* microorganisms

The host *B. subtilis* (BGSC strain 1A289) was initially both $Cm^S$ and deficient in the ability to make α-amylase ($Amy^-$).

A preferred amylase plate membrane detection assay was developed, which allowed the detection of a relatively small number of $Amy^+$ among a large population of $Amy^-$ cells.

An agar medium of TBAB, 0.1 percent casamino acids (Difco Laboratories, Detroit, Michigan) and 5 µg/ml chloramphenicol, incorporating 1.0 percent (w/v) starch, was prepared. A suitable starch is a starch "Specific for Diastatis Power and α-amylase Determination," available from American Society of Brewing Chemists, Inc., St. Paul, Minnesota 55121. Twenty-five ml portions of the medium were placed in 100 mm petri dishes (plates). The plates can be stored in sealed plastic sleeves at 4°C for up to 3 weeks with no deleterious effects.

Semipermeable membranes, having a 0.45 µm pore size, were used as follows. A suitable membrane is commercially available from Millipore Corporation, Bedford, Massachusetts, under the trade designation Hybridization Membrane Filter Discs.

Two cuts, approximately 5 mm in length were made at right angles to each other, to serve as orientation marks for positioning the membrane onto the starch agar plates. Ashless filter papers were cut into thin strips (approximately 5 mm×50 mm) and placed between the membranes during sterilization. The stacked membranes were submerged in water in a glass petri dish and autoclaved for 30 minutes at 120°C.

The sterile membranes were overlaid on the agar surface of the starch plates by aseptic transfer with sterile filter forceps. On a plate turntable, a sterile glass rod was used to remove the wrinkles and the air bubbles, which prevent intimate contact between the membrane and the agar surface. The positions of the nicks in the filter were labeled on the bottom of each plate. It is critical to the efficiency of the assay that the starch plates be fresh and free of bubbles to ensure that there is intimate contact of the membrane with all areas of the agar.

Following the procedure for plasmid transformation of *B. subtilis* host microorganisms, 0.1 ml of cells, containing approximately $1 \times 10^5$ colony-forming units per ml, were plated directly onto the filters. The plates were incubated at 37°C for 18-24 hours with the plate right-side up. After the incubation period, the membranes were aseptically removed with filter forceps. The membrane was removed and transferred to a sterile petri dish and appropriately labeled. In this way, the colonies themselves were not exposed to iodine vapor, which is lethal in high concentrations. Each starch-agar plate was held with gloves directly over the iodine vapor for 4-7 seconds, then placed on a light source to visualize the clear zones of starch hydrolysis against the blue starch-iodine background. One amylase-producing colony in a field of $1 \times 10^5$ $Amy^-$ colonies was detectable by this technique. The locations of the zones were marked directly on the plate bottom immediately after the plate was iodine-developed. This was necessary because the stain begins to fade after 7-15 seconds. In lieu of the iodine vapor step, 2 ml of an aqueous iodine solution (2 percent potassium iodide, 0.2 percent iodine) can be used to visualize the zones. Application of iodine in this manner results in a stain which is retained longer than by the vapor method.

In order to recover the amylase-producing colonies, the membrane containing the $Amy^+$ cells was identified and transferred back to its original starch-iodine agar plate using the nicks in the filter to achieve proper alignment. By holding the plate over a light source, the labeled zones could be seen through the filter and correlated with a colony on the filter.

The colony was removed from the filter by scraping with a sterile toothpick and suspending the cells in 1.0 ml of TBB broth (Tryptose Beef Broth: 1.0 percent tryptose, 0.3 percent beef extract, 0.5 percent

NaCl, and 0.1 percent casamino acids). Serial dilutions were made and 0.1 ml cells was plated onto filters over TBAB+starch+chloramphenicol plates as before. The filter detection assay, described above, was repeated until it was established that a pure culture of Amy⁺ Cm$^R$ B. subtilis cells had been obtained.

The pure culture was grown in broth culture and tested for α-amylase activity. The culture produced a product that showed the same electrophoretic mobility as α-amylase produced by the B. licheniformis microorganism from which the cloned gene was derived. These tests indicated that the method described was operable for cloning the α-amylase gene into the Bacillus host microorganism.

Cloning of the B. licheniformis α-amylase gene into a B. subtilis host was desirable for two reasons. First, B. licheniformis is not amenable to genetic manipulation by standard techniques. Therefore, the genetic basis for α-amylase production can be more easily studied in the well-characterized B. subtilis host. Second. B. subtilis may often be a more desirable fermentation organism than B. licheniformis for industrial applications.

Any suitable selection procedure for the selection of the transformed Bacillus host microorganisms can be used by those skilled in the art.

## Claims

1. A method for cloning and isolating a gene encoding an enzyme selected from the group consisting of α-amylase, protease, amyloglucosidase from a Bacillus microorganism into a Bacillus host microorganism of another species which comprises the steps of:

(a) digesting a bifunctional plasmid having the capability to replicate in both E. coli and B. subtilis, wherein said plasmid contains at least two selectable markers, with an endonuclease to produce a linear plasmid molecule;

(b) generating DNA fragments containing said gene and incorporating said DNA fragments into said plasmid of (a) such that insertional inactivation of one of said selectable markers to produce a non-functional marker occurs, leaving at least one functional selectable marker, to produce a mixture containing recombinant hybrid plasmids and as a by-product, said bifunctional plasmids of (a);

(c) incorporating said mixture of (b) into E. coli microorganisms which do not have said selectable markers of (a) to transform said E. coli;

(d) selecting E. coli transformants containing said plasmids of (b) by means of said functional selectable marker;

(e) identifying transformants containing said recombinant plasmids by screening said transformants of (d) for the presence of said nonfunctional insertionally-inactivated marker;

(f) extracting plasmid DNA from said E. coli transformants of (e);

(g) transforming said Bacillus host microorganisms which do not contain said selectable markers of (a) with said plasmid DNA from (f);

(h) selecting said Bacillus host transformants containing recombinant plasmids by means of said functional selectable marker;

(i) screening said Bacillus host microorganisms selected in step (h) by overlaying a sterile semipermeable membrane on the surface of an agar medium containing a substance which is capable of being reacted upon by said enzyme, growing said transformed microorganisms on said membrane and allowing said enzyme to pass into said agar while retaining said transformed microorganisms on said membrane, removing said membrane from said agar surface, and contacting said agar with a substance which allows detection of whether a reaction between the said enzyme and the said substance has taken place; and

(j) isolating therefrom a Bacillus host microorganism capable of producing said enzyme encoded by said gene on said plasmid.

2. A method as claimed in claim 1 wherein said selectable marker involves antibiotic resistance or a growth factor.

3. A method as claimed in claims 1 or 2 wherein the Bacillus host microorganism is selected from the group consisting of B. subtilis, B. licheniformis and B. amyloliquifaciens.

4. A method as claimed in claims 1-3 wherein the plasmid is pHV33 (ATCC 39217).

5. A method for cloning and isolating an α-amylase gene from a Bacillus microorganism into a Bacillus host microorganism of another species which comprises the steps of:

(a) digesting a bifunctional plasmid having the capability to replicate in both E. coli and B. subtilis wherein said plasmid contains at least two selectable markers, with an endonuclease to produce a linear plasmid molecule;

(b) generating DNA fragments containing said gene and incorporating said DNA fragments into said plasmid of (a) such that insertional inactivation of one of said selectable markers to produce a nonfunctional marker occurs, leaving at least one functional selectable marker, to produce a mixture containing recombinant hybrid plasmids and as a by-product, said bifunctional plasmids of (a);

(c) incorporating said mixture of (b) into *E. coli* microorganisms which do not have said selectable markers of (a) to transform said *E. coli*;

(d) selecting *E. coli* transformants containing said plasmids of (b) by means of said functional selectable marker;

(e) identifying transformants containing said recombinant plasmids by screening said transformants of (d) for the presence of said nonfunctional insertionally-inactivated marker;

(f) extracting plasmid DNA from said *E. coli* transformants of (e);

(g) transforming said *Bacillus* host microorganisms which do not contain said selectable markers of (a) with said plasmid DNA from (f);

(h) selecting said *Bacillus* host transformants containing recombinant plasmids by means of said functional selectable marker;

(i) screening said transformed *Bacillus* host microorganisms selected in step (h) by overlaying a sterile semipermeable membrane on the surface of an agar medium containing starch, growing said transformed microorganisms on said membrane and allowing said α-amylase to pass into said agar while retaining said transformed microorganisms on said membrane, removing said membrane from said agar surface, contacting said agar with an iodine-containing reagent and observing a detectable reaction between α-amylase and the starch-iodine; and

(j) isolating therefrom a *Bacillus* host microorganism capable of producing α-amylase.

6. A method as claimed in claim 5 wherein the host microorganism is selected from the group consisting of *B. substilis. B. licheniformis* and *B. amyloliquifaciens.*

7. A method as claimed in claims 5 or 6 wherein the plasmid is pHV33 (ATCC 39217).

8. A method as claimed in claims 5-7 wherein said selectable marker involves antibiotic resistance.

**Patentansprüche**

1. Verfahren zum Klonen une Isolieren eines Gens, das ein Enzym, ausgewählt aus der Reihe alpha-Amylase, Protease, Amyloglucosidase, kodiert, aus einem Bacillus-Mikroorganismus in einen Bacillus-Wirts-Mikroorganismus einer anderen Spezies, das die Schritte umfasst:

(a) Digerieren eines bifunktionellen Plasmids, das die Fähigkeit zur Replikation sowohl in E. coli als auch in B. subtilis besitzt, wobei das Plasmid mindestens zwei selektierbare Marker enthält, mit einer Endonuklease zur Herstellung eines linearen Plasmidmoleküls;

(b) Erzeugen von DNS-Bruchstücken, die das Gen enthalten, und Einfügen der DNS-Bruchstücke in das Plasmid von (a), so dass die Insertionsinaktivierung eines der selektierbaren Marker unter Erzeugen eines nichtfunktionellen Markers geschieht, Bestehenlassen mindestens eines funktionellen, selektierbaren Markers, um eine Mischung herzustellen, die rekombinante Hybridplasmide und als ein Nebenprodukt die bifunctionellen Plasmide von (a) enthält;

(c) Einfügen der Mischung von (b) in E. coli-Mikroorganismen, die keine selektierbaren Marker von (a) aufweisen, umd das E. coli au transformieren;

(d) Auswählen der E. coli-Transformanden, die die Plasmide von (b) enthalten, mittels des funktionellen, selektierbaren Markers;

(e) Identifizieren der Transformanden, die die rekombinanten Plasmide enthalten, durch Screening der Transformanden von (d) hinsichtlich des Vorhandenseins des nichtfunktionellen, insertionsinaktivierten Markers;

(f) Extrahieren der Plasmid-DNS aus den E. coli-Transformanden von (e);

(g) Transformieren der Bacillus-Wirts-Mikroorganismen, die keine selektierbaren Marker von (a) enthalten, mit der Plasmid-DNS von (f);

(h) Auswählen der Bacillus-Wirts-Transformanden, die rekombinante Plasmide enthalten, mittels des funktionellen, selektierbaren Markers;

(i) Screening der Bacillus-Wirts-Mikroorganismen, die in Schritt (h) augewählt wurden, durch überlagern einer sterilen, semipermeablen Membran auf die Oberfläche eines Agarmediums, das eine Substanz enthält, die in der Lage ist, mit dem Enzym umgesetzt zu werden, Züchten der transformierten Mikroorganismen auf der Membran und Übertragung des Enzyms in das Agar, während die transformierten Mikroorganismen auf der Membran zurückgehalten werden, Entfernen der Membran aus der Agaroberfläche und Kontaktieren des Agars mit einer Substanz, die nachweisen erlauben wenn eine reaktion zwischen dem Enzym und der Substanz stattgefunden hat; und

(j) daraus Isolieren eines Bacillus-Wirts-Mikroorganismus, der in der Lage ist, das Enzym, das durch das Gen auf dem Plasmid kodiert ist, zu erzeugen.

2. Verfahren nach Anspruch 1, wobei der selektierbare Marker eine antibiotische Widerstandsfähigkeit oder einen Wachstumsfaktor einschliesst.

3. Verfahren nach Anspruch 1 oder 2, wobei der Bacillus-Wirts-Mikroorganismus aus der Reihe B. subtilis, B. licheniformis und B. amyloliquefaciens ausgewählt wird.

4. Verfahren nach Anspruch 1 bis 3, wobei das Plasmid pHV33 (ATCC 39217) ist.

5. Verfahren zum Klonen und Isolieren eines alpha-Amylase-Gens aus einem Bacillus-Mikroorganismus in einen Bacillus-Wirts-Mikroorganismus einer anderen Spezies, das die Schritte umfasst :

   (a) Digerieren eines bifunctionellen Plasmids, das die Fähigkeit zur Replikation sowohl in E. coli als auch in B. subtilis besitzt, wobei das Plasmid mindestens zwei selektierbare Marker enthält, mit einer Endonuklease, um ein lineares Plasmidmolekül zu erzeugen;
   (b) Erzeugen von DNS-Bruchstücken, die das Gen enthalten, und Einfügen der DNS-Bruchstücke in das Plasmid von (a), so dass eine Insertionsinaktivierung eines der selektierbaren Marker unter Herstellung eines nichtfunktionelles Markers auftritt, Bestehenlassen mindestens eines funktionellen, selektierbaren Markers, um eine Mischung zu erzeugen, die rekombinante Hybridplasmide und als ein Nebenprodukt die bifunktionellen Plasmide von (a) enthält;
   (c) Einfügen der Mischung von (b) in E. coli-Mikroorganismen, die keine selektierbaren Marker von (a) aufweisen, um das E. coli zu transformieren;
   (d) Auswählen der E. coli-Transformanden, die die Plasmide von (b) enthalten, mittels des funktionellen, selektierbaren Markers;
   (e) Identifizieren des Transformanden, die die rekombinanten Plasmide enthalten, durch Screening der Transformanden von (d) hinsichtlich des Vorhandenseins des nichtfunktionellen, insertionsinaktivierten Markers;
   (f) Extrahieren von Plasmid-DNS aus den E. coli-Transformanden von (e);
   (g) Transformieren der Bacillus-Wirts-Mikroorganismen, die keine selektierbaren Marker von (a) aufweisen, mit dem Plasmid-DNS aus (f);
   (h) Auswhl der Bacillus-Wirts-Transformanden, die rekombinante Plasmide enthalten, mittels des funktionellen, selektierbaren Markers;
   (i) Screening der transformierten Bacillus-Wirts-Mikroorganismen, die in Schritt (h) ausgewählt wurden, durch Überlagern einer sterilen, semipermeablen Membran auf die oberfläche eines Agarmediums, das Stärke enthält Züchten der transformierten Mikroorganismen auf der Membran und Übertragen von alpha-Amylase in das Agar, während die transformierten Mikroorganismen auf der Membran zurückgehalten werden, Entfernen der membran aus der Agaroberfläche, Kontaktieren des Agars mit einem jodhaltigen Reagens und Beobachten einer Nachweisreaktion zwischen alpha-Amylase und Stärke-Jod; und
   (j) daraus Isolieren eines Bacillus-Wirts-Mikroorganismus, der in der Lage ist, alpha-Amylase herzustellen.

6. Verfahren nach Anspruch 5, wobei der Wirts-Mikroorganismus aus der Reihe B. subtilis, B. licheniformis und B. amyloliquefaciens ausgewählt wird.

7. Verfahren nach Anspruch 5 oder 6, wobei das Plasmid pHV33 (ATCC 39217) ist.

8. Verfahren nach Anspruch 5 bis 7, wobei der selektierbare Marker eine antibiotische Widerstandsfähigkeit einschliesst.

**Revendications**

1. Procédé pour cloner et isoler un gène codant pour une enzyme, choisie parmi une α-amylase, une protéase, une amyloglucosidase, d'un microorganisme Bacillus dans un microorganisme Bacillus hôte d'une autre espèce, qui comprend les stades de :

   (a) digestion d'un plasmide bifonctionnel ayant la capacité de se répliquer dans E. coli et dans B. subtilis, ledit plasmide contenant en moins deux marqueurs sélectionnables, avec une endonucléase pour produire une molécule plasmidique linéaire;
   (b) formation de fragments d'ADN contenant ledit gène et incorporation desdits fragments d'ADN dans ledit plasmide de (a), de façon à effectuer une inactivation par insertion d'un desdits marqueurs sélectionnables pour produire un marqueur non fonctionnel, en laissant au moins un marqueur sélectionnable fonctionnel, pour produire un mélange contenant des plasmides hybrides recombinants et, comme sous-produit, lesdits plasmides bifonctionnels de (a);
   (c) incorporation dudit mélange de (b) dans des microorganismes E. coli qui n'ont pas lesdits marqueurs sélectionnables de (a) pour transformer lesdits E. coli;

(d) sélection des transformants d'E. coli contenant lesdits plasmides (b) à l'aide dudit marqueur sélectionnable fonctionnel;

(e) identification des transformants contenant lesdits plasmides recombinants par criblage desdits transformants de (d) relativement à la présence dudit marqueur non fonctionnel inactivé par insertion;

(f) extraction de l'ADN plasmidique desdits transformants d'E. coli de (e);

(g) transformation desdits microorganismes Bacillus hôtes qui ne contiennent pas lesdits marqueurs sélectionnables de (a) avec ledit ADN plasmidique de (f);

(h) sélection desdits transformants Bacillus hôtes contenant les plasmides recombinants à l'aide dudit marqueur sélectionnable fonctionnel;

(i) criblage desdits microorganismes Bacillus hôtes sélectionnés dans le stade (h) par recouvrement avec une membrane semi-perméable stérile de la surface d'un milieu gélosé contenant une substance avec laquelle ladite enzyme peut réagir, culture desdits microorganismes transformés sur ladite membrane en laissant passer ladite enzyme dans ladite gélose, tout en retenant lesdits microorganismes transformés sur ladite membrane, séparation de ladite membrane de ladite surface de gélose et contact de ladite gélose avec une substance qui permet de détecter si une réaction a eu lieu entre ladite enzyme et ladite substance; et

(j) isolement d'un microorganisme Bacillus hôte capable de produire ladite enzyme codée par ledit gène sur ledit plasmide.

2. Procédé selon la revendication 1 dans lequel ledit marqueur sélectionnable fait intervenir une résistance à un antibiotique ou un facteur de croissance.

3. Procédé selon la revendication 1 ou 2, dans lequel le microorganisme Bacillus hôte est choisi dans le groupe constitué de B. subtilis., B. licheniformis et B. amyloliquefaciens.

4. Procédé selon les revendications 1 à 3 dans lequel le plasmide est pHV33 (ATCC 39217).

5. Procédé pour cloner et isoler un gène d'$\alpha$-amylase à partir d'un microorganisme Bacillus dans un microorganisme Bacillus hôte d'une autre espèce, qui comprend les stades de :

(a) digestion d'un plasmide bifonctionnel ayant la capacité de se répliquer dans E. coli et dans B. subtilis, ledit plasmide contenant au moins deux marqueurs sélectionnables, avec une endonucléase pour produire une molécule plasmidique linéaire;

(b) formation de fragments d'ADN contenant ledit gène et incorporation desdits fragments d'ADN dans ledit plasmide de (a) de façon à effectuer une inactivation par insertion d'un desdits marqueurs sélectionnables pour produire un marqueur non fonctionnel, en laissant au moins un marqueur sélectionnable fonctionnel, pour produire un mélange contenant des plasmides hybrides recombinants et, comme sous-produit, lesdits plasmides bifonctionnels de (a);

(c) incorporation dudit mélange de (b) dans des microorganismes E. coli qui n'ont pas lesdits marqueurs sélectionnables de (a) pour transformer lesdits E. coli;

(d) sélection des transformants d'E. coli contenant lesdits plasmides (b) à l'aide dudit marqueur sélectionnable fonctionnel;

(e) identification des transformants contenant lesdits plasmides recombinants par criblage desdits transformants de (d) relativement à la présence dudit marqueur non fonctionnel inactivé par insertion;

(f) extraction de l'ADN plasmidique desdits transformants d'E. coli de (e);

(g) transformation desdits microorganismes Bacillus hôtes qui ne contiennent pas lesdits marqueurs sélectionnables de (a) avec ledit ADN plasmidique de (f);

(h) sélection desdits transformants Bacillus hôtes contenant les plasmides recombinants à l'aide dudit marqueur sélectionnable fonctionnel;

(i) criblage desdits microorganismes Bacillus hôtes transformés, sélectionnés dans le stade (h), par recouvrement avec une membrane semi-perméable stérile de la surface d'un milieu gélosé contenant de l'amidon, culture desdits microorganismes transformés sur ladite membrane en laissant ladite $\alpha$-amylase passer dans ladite gélose en retenant lesdits microorganismes transformés sur ladite membrane, séparation de ladite membrane de ladite surface de gélose, contact de ladite gélose avec un réactif contenant de l'iode et observation d'une réaction détectable entre l'$\alpha$-amylase et l'amidoniode; et

(j) isolement d'un microorganisme Bacillus hôte capable de produire de l'$\alpha$-amylase.

6. Procédé selon la revendication 5, dans lequel le microorganisme hôte est choisi dans le groupe constitué de B. subtilis, B. licheniformis et B.

amyloliquefaciens.

7. Procédé selon la revendication 5 ou 6 dans lequel le plasmide est pHV33 (ATCC 39217).

8. Procédé selon les revendications 5 à 7, dans lequel ledit marqueur détectable fait intervenir une résistance à un antibiotique.